# EUROPEAN PATENT APPLICATION

(11) **EP 4 325 213 A1**
(43) Date of publication of application: **21.02.2024**
(21) Application number: 22191061.5
(22) Date of filing: 18.08.2022
(51) Int. Cl.: G01N 27/12

(54) **A CHEMO-RESISTIVE GAS SENSING DEVICE COMPRISING A CATALYTIC GAS FILTER ARRANGEMENT**

(71) Applicant: Infineon Technologies AG, 85579 Neubiberg (DE)
(72) Inventor: KRIVEC, Matic, 9504 Villach-Warmbad (AT); ZOEPFL, Alexander, 93049 Regensburg (DE); ROTH, Alexandra Marina, 92318 Neumarkt (DE); MAIER, Dominic, 92714 Pleystein (DE); MEYER, Markus, 93161 Sinzing (DE); BREUER, Werner, 93161 Sinzing-Viehhausen (DE)
(74) Representative: Hersina, Günter

(57) **Abstract**

Disclosed is a gas sensing device for sensing one or more gases to be sensed in an ambient mixture of a plurality of gases, The gas sensing device comprises:
a sensor chip comprising a substrate and one or more chemo-resistive gas sensing elements attached to the substrate, wherein each of the gas sensing elements is configured for providing a sensor signal depending on at least one gas of the one or more gases to be sensed; and
a catalytic gas filter arrangement comprising one or more filter sections, wherein each of the filter sections comprises a cavity which is covered with at least one membrane, wherein the at least one membrane is supported by a support structure, wherein the at least one membrane comprises gas permeable pores, wherein a surface defining the pores comprises a catalytic material for degrading at least one of the gases of the plurality of the gases;
wherein the gas filter arrangement is arranged in such way that at least one of the chemo-resistive gas sensing elements is exposed to a filtered mixture of the plurality of gases in the cavity of one of the filter sections, wherein the filtered mixture of the plurality of gases is obtained by filtering the ambient mixture of the plurality of gases with the one of the filter sections.

## Description

### Technical Field

Embodiments relate to a gas sensing device for sensing one or more gases in a mixture of a plurality of gases. More particular, the disclosure deals with the estimation of gas concentrations through the use of chemo-resistive gas sensors.

### Background of the Invention

The sensing mechanisms of chemo-resistive sensors such as metal oxide (MOX) or graphene-based ones enables sensitive detection of many environmental gases, such as nitrogen oxide (NOx), ozone (O3), ethanol (C2H60), volatile organic compounds (VOCs). In principle, the gases need to adsorb on the active sensor material and either undergo chemical reactions, such as oxidation/reduction of MOX materials, or change the local electronic state of materials like graphene, which leads to a measurable signal change. However, such sensing mechanisms often lack selectivity in real conditions, i.e. in presence of gas mixtures. If, for example, the gas mixture has a high concentration of O3 and one of the gases to be sensed is NO2, the sensor response to O3 may be predominant and may shadow the much lower response to NO2 so that it is impossible to effectively and precisely predict the concentration of NO2.

### Summary of the Invention

Disclosed is a gas sensing device for sensing one or more gases to be sensed in an ambient mixture of a plurality of gases, The gas sensing device comprises:
a sensor chip comprising a substrate and one or more chemo-resistive gas sensing elements attached to the substrate, wherein each of the gas sensing elements is configured for providing a sensor signal depending on at least one gas of the one or more gases to be sensed; and
a catalytic gas filter arrangement comprising one or more filter sections, wherein each of the filter sections comprises a cavity which is covered with at least one membrane, wherein the at least one membrane is supported by a support structure, wherein the at least one membrane comprises gas permeable pores, wherein a surface defining the pores comprises a catalytic material for degrading at least one of the gases of the plurality of the gases;
wherein the gas filter arrangement is arranged in such way that at least one of the chemo-resistive gas sensing elements is exposed to a filtered mixture of the plurality of gases in the cavity of one of the filter sections, wherein the filtered mixture of the plurality of gases is obtained by filtering the ambient mixture of the plurality of gases with the one of the filter sections.

The one or more chemo-resistive gas sensing elements may be graphene gas sensing elements or reduced graphene gas sensing elements, where the base material is functionalized with specific chemicals, e.g. with platinum (Pt), or manganese dioxide (MnO2), so that each of the gas sensors is sensitive for gases, e.g. for nitrogen dioxide (NO2), ozone (O3) or carbon monoxide (CO). In doing so, the interaction between graphene sheets and absorbed gas analytes influences the electronic structure of the material depending on the mixture of gases, resulting in altered charge carrier concentration and changed electrical conductance. The one or more chemo-resistive gas sensing elements may also be metal oxide sensing elements which undergo, in the presence of gas analytes, chemical reactions, such as oxidation/reduction, so that their electrical properties change depending on the interacting gases.

In case of multi-gas sensing a multi-gas sensor array comprising a plurality of chemo-resistive gas sensors having dissimilar selectivity may be used. Due to the different sensitivity towards various gas molecules, resistances of the gas sensors change in disparate patterns, making it possible to analyze complicated gas mixtures with one single sensor array.

The sensor signals are typically electrical signals, which refer to electrical properties of the sensing material of one of the gas sensors, which depend on the interacting gases. The electrical properties may include the electrical resistance or the electrical conductivity of the sensing material. The signals may be time-discrete signals or continuous-time signals.

The main idea of this disclosure is to use catalytic materials (metals or oxides e.g. Ni, Ti, Cu, TiO₂, CuO, WO₃, etc.) for selective, continuous, in-situ degradation of environmental gases, such as O3 at or inside the gas sensing device. The catalytic material may degrade the molecules of a shadowing gas, for example, molecules of O3, locally in proximity of the chemo-resistive gas sensing elements or elements and whereby decrease the concentration of the shadowing gas and increase the prediction sensitivity of a gas to be sensed, such as NO2.

The disclosure overcomes above-mentioned drawbacks by ensuring that a shadowing gas or a plurality of shadowing gases does not reach the chemo-resistive gas sensing elements or elements or that the shadowing gas or the plurality of shadowing gases reaches the chemo-resistive gas sensing elements or elements at least in smaller amounts.

The substrate of the sensor chip may be made of a semi-conductor material.

The one or more chemo-resistive gas sensing elements may be graphene gas sensing elements or reduced graphene gas sensing elements, where the base material is functionalized with specific chemicals, e.g. with platinum (Pt), or manganese dioxide (MnO2), so that each of the gas sensors is sensitive for gases, e.g. for nitrogen dioxide (NO2), ozone (O3) or carbon monoxide (CO). In doing so, the interaction between graphene sheets and absorbed gas analytes influences the electronic structure of the material depending on the mixture of gases, resulting in altered charge carrier concentration and changed electrical conductance. The one or more chemo-resistive gas sensing elements may also be metal oxide sensing elements which undergo, in the presence of gas analytes, chemical reactions, such as oxidation/reduction, so that their electrical properties change depending on the interacting gases.

In case of multi-gas sensing a multi-gas sensor array comprising a plurality of chemo-resistive gas sensors having dissimilar selectivity may be used. Due to the different sensitivity towards various gas molecules, resistances of the gas sensors change in disparate patterns, making it possible to analyze complicated gas mixtures with one single sensor array.

The sensor signals are typically electrical signals, which refer to electrical properties of the sensing material of one of the gas sensors, which depend on the interacting gases. The electrical properties may include the electrical resistance or the electrical conductivity of the sensing material. The signals may be time-discrete signals or continuous-time signals.

The pores of the membrane serve as an inlet of the cavity for the plurality of gases, wherein at least one of the gases is degraded when passing the pores so that it does not reach the one or more chemo-resistive gas sensing elements within the cavity of the respective filter section.

The geometry and dimensions of the membrane and its pores are mandatory for its function. It has to be ensured that the targeted gas species/molecule get in interaction with the catalytic material. If the pore size is too big and diffusion length is too short, there can be no function. Moreover, the gas diffusion through such a filter can be controlled by the dimensions. The relevant dimensions are for example the pore diameter, the cross-sectional area of the pores, the perimeter of the pores, the length of the pores and the number of the pores.

The catalytic material or the catalytic materials may be selected depending on the specific use case different catalytic materials have different adsorption and oxidation properties and degradation kinetics for different gases. Focusing on O3 decomposition, several metals including platinum (Pt), palladium (Pd), rhodium (Rh), cerium (Ce), manganese (Mn), cobalt (Co), iron (Fe), nickel (Ni), zinc (Zn), silver (Ag), copper (Cu) and metal oxides including titanium dioxide (Ti02), cupric oxide (CuO), ferric oxide (Fe2O3), nickel oxide (NiO) have been reported to have efficient catalytic activity for O3 decomposition [1].

The proposed solution is superior to the use of traditional adsorption filters, as filters based on molecule adsorption, comprising for example activated carbon, can probably provide selective filtration to a certain degree based on different adsorption energies for different gases, but are prone to saturating and losing their activity after a certain time so that they need to be exchanged. The use of chemical reaction (e.g. O3 reaction with indigo trisulphonate) would be efficient in terms of selectivity, but would consume stoichiometrical amount of reactant and cannot be used continuously.

Moreover, the catalytic gas filter arrangement is able to work continuously and the materials used are stable and are not consumed during operation. In addition, the catalytic gas filter arrangement is highly selective.

According to embodiments of the disclosure, the at least one membrane of one or more of the one or more filter sections comprise a semiconductor bearing layer being coated with the catalytic material. The semiconductor bearing layer may be made using existing technologies. In particular, the semiconductor bearing layer can be a thin diffuser made of polyamide which is coated via physical vapor deposition (PVD) with a thin layer of metal or metal oxide as the catalytic material. By these features, the needed amount of the catalytic material may be minimized. In other embodiments, the membrane is completely made of the catalytic material.

According to embodiments of the disclosure, the support structure of one of the filter sections comprises one or more layers of the sensor chip, so that the cavity of the one of the filter sections is defined by the sensor chip and by the at least one membrane of the one of the filter sections. By such features, no support structure separate from the sensor chip is needed.

According to embodiments of the disclosure, at least the support structure of one of the filter sections comprises a plate-shaped semiconductor chip having a through hole. Such support structures are easy to manufacture. They may have one through hole for defining the cavity of one filter section or they may have a plurality of through holes for defining the cavities of a plurality of filter sections.

According to embodiments of the disclosure, the plate-shaped semiconductor chip of one of the filter sections is attached to the sensor chip, so that the cavity of the one of the filter sections is defined by the through hole of the plate-shaped semiconductor chip, by the sensor chip and by the at least one membrane of the one of the filter sections. Such embodiments allow miniaturization of the gas sensing device.

According to embodiments of the disclosure, the plate-shaped semiconductor chip of one of the filter sections is attached to a housing of the gas sensing device, so that the cavity of the one of the filter sections is defined by the housing and by the at least one membrane of the one of the filter sections. Such embodiments allow to use membranes having a large amount of pores so that a higher flow of the gases into the cavity can be achieved, which results in a more dynamic behavior of the gas sensing device.

According to embodiments of the disclosure, the gas sensing device comprise one or more particle filters for preventing a penetration of particles into the cavity of one or more of the one or more filter sections. Such features prevent clogging of the one or more chemo-resistive gas sensing elements of the respective filter section. In case that the particle filters is arranged at a side of the membrane of the respective filter section, which faces away from the cavity of the respective filter section, the particle filter also may prevent of clogging of the membrane.

According to embodiments of the disclosure, the one or more particle filters are attached to a housing of the gas sensing device. Such embodiments allow to use particle filters having a large active cross-sectional area so that a higher flow of the gases into the cavity can be achieved, which results in a more dynamic behavior of the gas sensing device.

According to embodiments of the disclosure, the sensor chip comprises a first chemo-resistive gas sensing element of the one or more chemo-resistive gas sensing elements and a second chemo-resistive gas sensing element of the one or more chemo-resistive gas sensing elements, wherein the catalytic gas filter arrangement comprises a first filter section of the one or more filter sections and a second filter section of the one or more filter sections, wherein the first filter section is arranged for filtering the ambient mixture of the plurality of gases for the first chemo-resistive gas sensing element, and wherein the second filter section is arranged for filtering the ambient mixture of the plurality of gases for the second chemo-resistive gas sensing element. By such features, the selectivity of the gas sensing device with respect to different gases may be increased.

According to embodiments of the disclosure, a sensing material of the first chemo-resistive gas sensing element is different from a sensing material of the second chemo-resistive gas sensing element. By such features, the selectivity of the gas sensing device with respect to different gases may be increased.

According to embodiments of the disclosure, the catalytic material of the first filter section is different from the catalytic material of the second filter section. By such features, the selectivity of the gas sensing device with respect to different gases may be increased.

According to embodiments of the disclosure, the sensor chip comprises a fifth chemo-resistive gas sensing element of the one or more chemo-resistive gas sensing elements and a sixth chemo-resistive gas sensing element of the one or more chemo-resistive gas sensing elements, wherein the catalytic gas filter arrangement comprises a fifth filter section of the one or more filter sections, wherein the fifth filter section is arranged for filtering the ambient mixture of the plurality of gases for the fifth chemo-resistive gas sensing element and for the sixth chemo-resistive gas sensing element. By such features, the selectivity of the gas sensing device with respect to different gases may be increased.

According to embodiments of the disclosure, a sensing material of the fifth chemo-resistive gas sensing element is different from a sensing material of the sixth chemo-resistive gas sensing element. By such features, the selectivity of the gas sensing device with respect to different gases may be increased.

According to embodiments of the disclosure, in a cross-sectional view the pores of the one or more membranes have a maximum extension smaller than 1000 nm and a minimum extension greater than 20 nm. Such dimensions of the pores ensure that the targeted gas species/molecule get in interaction with the catalytic material so that the selectivity of the gas sensing device with respect to different gases may be increased.

According to embodiments of the disclosure, in a longitudinal view the pores of the one or more membranes have a length smaller than 2000 nm and greater than 40 nm. Such dimensions of the pores ensure that the targeted gas species/molecule get in interaction with the catalytic material so that the selectivity of the gas sensing device with respect to different gases may be increased.

Preferred embodiments of the invention are subsequently discussed with respect to the accompanying drawings, in which:
- Figure 1: illustrates a first embodiment of a gas sensing device according to the invention in a schematic sectional side view;
- Figure 2: illustrates the first embodiment of a gas sensing device according to the invention in a schematic top view;
- Figure 3: illustrates a second embodiment of a gas sensing device according to the invention in a schematic sectional side view;
- Figure 4: illustrates the second embodiment of a gas sensing device according to the invention in a schematic top view;
- Figure 5: illustrates a third embodiment of a gas sensing device according to the invention in a schematic spatial view;
- Figure 6: illustrates a fourth embodiment of a gas sensing device according to the invention in a schematic spatial view;
- Figure 7: illustrates a fifth embodiment of a gas sensing device according to the invention in a schematic spatial view;
- Figure 8: illustrates a sixth embodiment of a gas sensing device according to the invention in a schematic sectional side view;
- Figure 9: illustrates the sixth embodiment of a gas sensing device according to the invention in a schematic top view;
- Figure 10: illustrates a seventh embodiment of a gas sensing device according to the invention in a schematic sectional side view;
- Figure 11: illustrates the seventh embodiment of a gas sensing device according to the invention in a schematic top view;
- Figure 12: illustrates an eighth embodiment of a gas sensing device according to the invention in a schematic sectional side view;
- Figure 13: illustrates the eighth embodiment of a gas sensing device according to the invention in a schematic top view, and
- Figure 14: illustrate the catalytic effects of different catalytic materials.

Equal or equivalent elements or elements with equal or equivalent functionality are denoted in the following description by equal or equivalent reference numerals.

In the following description, a plurality of details is set forth to provide a more thorough explanation of embodiments of the present disclosure. However, it will be apparent to those skilled in the art that embodiments of the present disclosure may be practiced without these specific details. In other instances, well-known structures and devices are shown in block diagram form rather than in detail in order to avoid obscuring embodiments of the present disclosure. In addition, features of the different embodiments described hereinafter may be combined with each other, unless specifically noted otherwise.

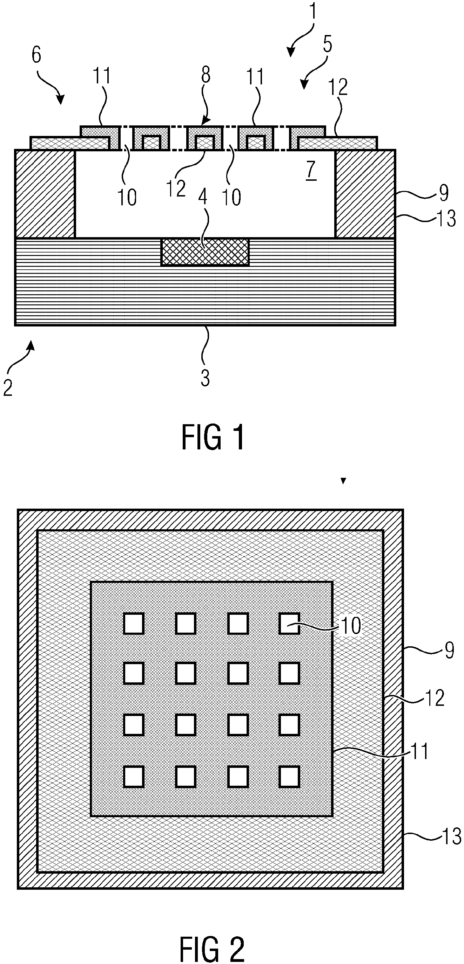

Figure 1 illustrates a first embodiment of a gas sensing device according to the invention in a schematic sectional side view.

Figure 2 illustrates the first embodiment of a gas sensing device according to the invention in a schematic top view.

The gas sensing device 1 according to the first embodiment is configured for sensing one or more gases to be sensed in an ambient mixture of a plurality of gases. The gas sensing device 1 comprises:
a sensor chip 2 comprising a substrate 3 and one or more chemo-resistive gas sensing elements 4 attached to the substrate 3, wherein each of the gas sensing elements 4 is configured for providing a sensor signal depending on at least one gas of the one or more gases to be sensed; and
a catalytic gas filter arrangement 5 comprising one or more filter sections 6, wherein each of the filter sections 6 comprises a cavity 7 which is covered with at least one membrane 8, wherein the at least one membrane 8 is supported by a support structure 9, wherein the at least one membrane 8 comprises gas permeable pores 10, wherein a surface defining the pores 10 comprises a catalytic material 11 for degrading at least one of the gases of the plurality of the gases;
wherein the gas filter arrangement 5 is arranged in such way that at least one of the chemo-resistive gas sensing elements 4 is exposed to a filtered mixture of the plurality of gases in the cavity 7 of one of the filter sections 6, wherein the filtered mixture of the plurality of gases is obtained by filtering the ambient mixture of the plurality of gases with the one of the filter sections 6.

According to embodiments of the disclosure, the at least one membrane 8 of one or more of the one or more filter sections 6 comprise a semiconductor bearing layer 12 being coated with the catalytic material 11.

According to embodiments of the disclosure, the support structure 9 of one of the filter sections 6 comprises one or more layers 13 of the sensor chip 2, so that the cavity 7 of the one of the filter sections is defined by the sensor chip 2 and by the at least one membrane 7 of the one of the filter sections 6.

According to embodiments of the disclosure, in a cross-sectional view the pores 10 of the one or more membranes 8 have a maximum extension smaller than 1000 nm and greater than 20 nm.

According to embodiments of the disclosure, in a longitudinal view the pores 10 of the one or more membranes 8 have a length smaller than 2000 nm and greater than 40 nm.

The gas sensing device 1 shown in Figures 1 and 2 comprises a sensor chip 2, which comprises a substrate 3 with one chemo-resistive gas sensing element 4. The chemo-resistive gas sensing element 4 is arranged in the cavity 7 of the sole filter section 6 of the catalytic gas filter arrangement 5. The support structure 9 is a layer 13 of the sensor chip 2. The cavity 7 is defined by the substrate 3, the gas sensing element 4, the layer 13 of the sensor chip 2 and the membrane 8. The membrane 8 comprises a semiconductor bearing layer 12, which is coated with a catalytic material 11, so that a surface defining the pores 10 of the membrane 8 is formed by the catalytic material 11

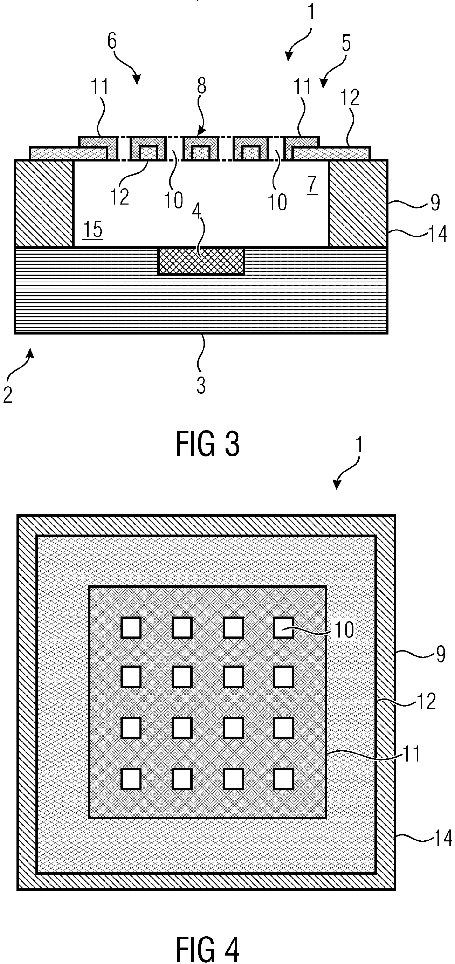

Figure 3 illustrates a second embodiment of a gas sensing device according to the invention in a schematic sectional side view.

Figure 4 illustrates the second embodiment of a gas sensing device according to the invention in a schematic top view.

The second embodiment of the gas sensing device 1 is based on the first embodiment of the gas sensing device 1. In the following, only the different features of the second embodiment of the gas sensing device 1 are discussed.

According to embodiments of the disclosure, at least the support structure 9 of one of the filter sections 6 comprises a plate-shaped semiconductor chip 14 having a through hole 15.

According to embodiments of the disclosure, the plate-shaped semiconductor chip 14 of one of the filter sections 6 is attached to the sensor chip 2, so that the cavity 7 of the one of the filter sections 6 is defined by the through hole 15 of the plate-shaped semiconductor chip 14, by the sensor chip 2 and by the at least one membrane 8 of the one of the filter sections 6.

At the gas sensing device 1 shown in Figures 3 and 4, the support structure 9 is a plate-shaped semiconductor chip 14 having a through hole 15. The plate-shaped semiconductor chip 14 is attached to the sensor chip 2, so that the cavity 7 is defined by the substrate 3, the gas sensing element 4, the plate-shaped semiconductor chip 14 and the membrane 8.

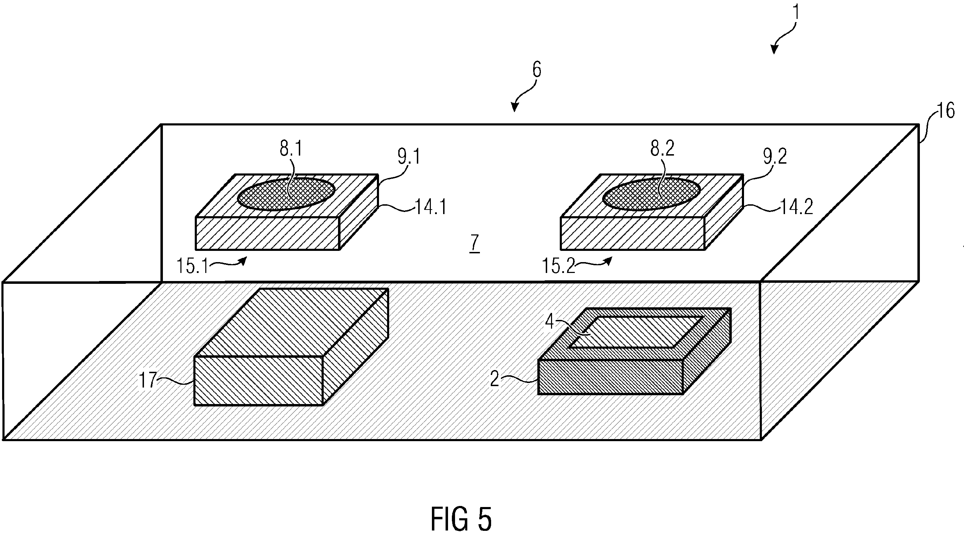

Figure 5 illustrates a third embodiment of a gas sensing device according to the invention in a schematic spatial view.

The third embodiment of the gas sensing device 1 is based on the second embodiment of the gas sensing device 1. In the following, only the different features of the third embodiment of the gas sensing device 1 are discussed.

According to embodiments of the disclosure, the plate-shaped semiconductor chip 14 of one of the filter sections 6 is attached to a housing 16 of the gas sensing device 1, so that the cavity 7 of the one of the filter sections 6 is defined by the housing 16 and by the at least one membrane 8 of the one of the filter sections 6.

The gas sensing device 1 shown in Figure 5 comprises a first membrane 8.1 and a second membrane 8.2. The first membrane 8.1 is supported by a first support structure 9.1, which is a first plate-shaped semiconductor chip 14.1, which has a first through hole 15.1. The second membrane 8.2 is supported by a second support structure 9.2, which is a second plate-shaped semiconductor chip 14.2, which has a first through hole 15.2. In other embodiments not shown, a single plate-shaped semiconductor chip having two through holes is used for supporting a first membrane and a second membrane.

The gas sensing device 1 shown in Figure 5 comprises one filter section 6, which has one cavity 7, which is defined by a housing 16 of the gas sensing device 1 as well as by the first membrane 8.1 and the second membrane 8.2. The first plate-shaped semiconductor chip 14.1 and the second plate-shaped semiconductor chip 14.2 are attached to the housing 16.

The housing also may contain an electronic control unit 17 connected to the sensing element 4 of the sensor chip 2. The electronic control unit 17 may be configured for determining the resistance of the active sensor material of the gas sensing element 4. For the same purpose, the electronic control unit 17 could be connected to a plurality of sensing elements 4 of the sensor chip 2.

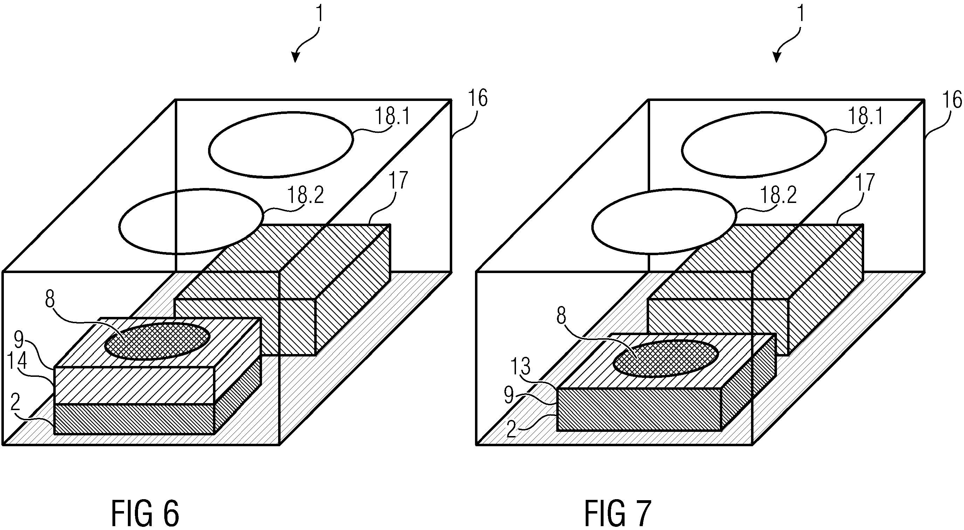

Figure 6 illustrates a fourth embodiment of a gas sensing device according to the invention in a schematic spatial view.

The fourth embodiment of the gas sensing device 1 is based on the second embodiment of the gas sensing device 1. In the following, only the different features of the fourth embodiment of the gas sensing device 1 are discussed.

According to embodiments of the disclosure, the gas sensing device 1 comprise one or more particle filters 18 for preventing a penetration of particles into the cavity 7 of one or more of the one or more filter sections 6.

According to embodiments of the disclosure, the one or more particle filters 18 are attached to a housing 16 of the gas sensing device 1.

The gas sensing device 1 shown in Figure 6 comprises a housing 16, which houses the sensor chip 2, the plate-shaped semiconductor chip 14 and the membrane 8 as well as the electronic control unit 17. A first particle filter 18.1 and a second particle filter 18.2 are attached to the housing 16, so that the gases may diffuse into the housing 16 but particles a prevented from doing so. By these features the membrane 8 and the gas sensing element of the sensor chip 2 are prevented from clogging by particles.

Figure 7 illustrates a fifth embodiment of a gas sensing device according to the invention in a schematic spatial view.

The fifth embodiment of the gas sensing device 1 is based on the first and the fourth embodiment of the gas sensing device 1. In the following, only the different features of the fifth embodiment of the gas sensing device 1 are discussed.

The gas sensing device 1 shown in Figure 7 comprises a housing 16, which houses the sensor chip 2 including the layer 13 and the membrane 8 as well as the electronic control unit 17. A first particle filter 18.1 and a second particle filter 18.2 are attached to the housing 16, so that the gases may diffuse into the housing 16 but particles a prevented from doing so. By these features the membrane 8 and the gas sensing element of the sensor chip 2 are prevented from clogging by particles.

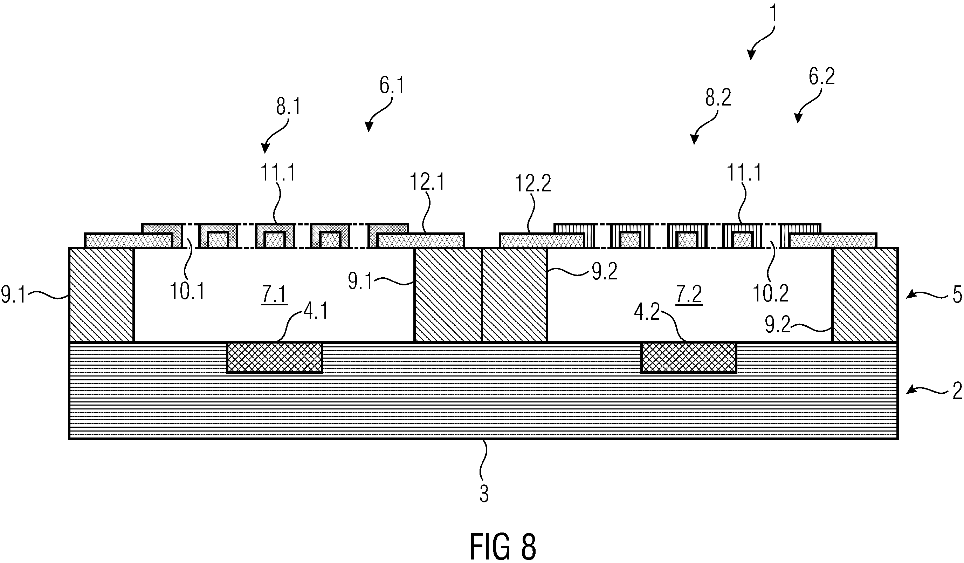

Figure 8 illustrates a sixth embodiment of a gas sensing device according to the invention in a schematic sectional side view.

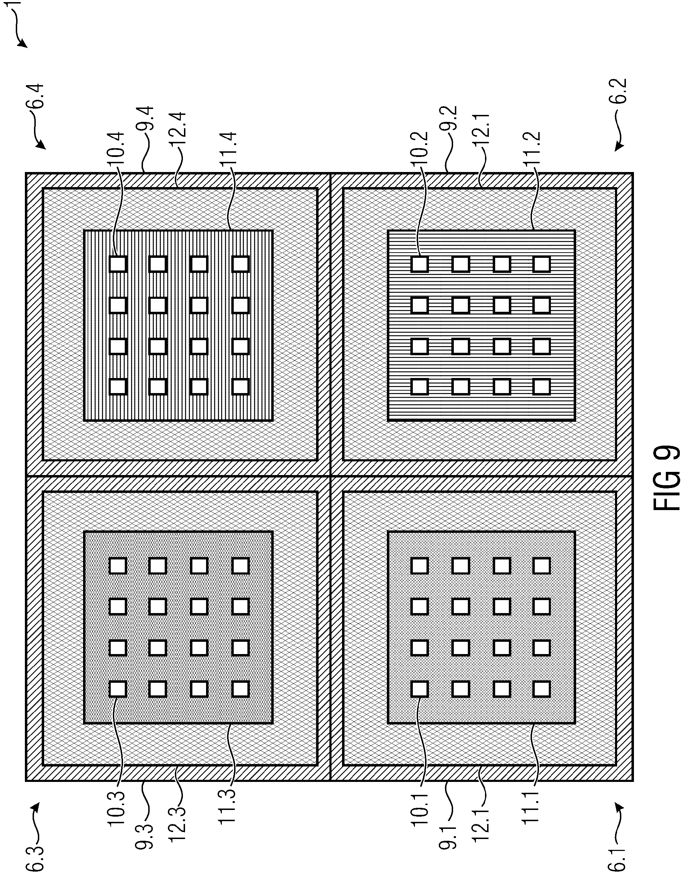

Figure 9 illustrates the sixth embodiment of a gas sensing device according to the invention in a schematic top view.

The sixth embodiment of the gas sensing device 1 is based on the first embodiment of the gas sensing device 1. In the following, only the different features of the sixth embodiment of the gas sensing device 1 are discussed.

According to embodiments of the disclosure, the sensor chip 2 comprises a first chemo-resistive gas sensing element 4.1 of the one or more chemo-resistive gas sensing elements 4 and a second chemo-resistive gas sensing element 4.2 of the one or more chemo-resistive gas sensing elements 4, wherein the catalytic gas filter arrangement 5 comprises a first filter section 6.1 of the one or more filter sections 6 and a second filter section 6.2 of the one or more filter sections 6, wherein the first filter section 6.1 is arranged for filtering the ambient mixture of the plurality of gases for the first chemo-resistive gas sensing element 4.1, and wherein the second filter section 6.2 is arranged for filtering the ambient mixture of the plurality of gases for the second chemo-resistive gas sensing element 4.

According to embodiments of the disclosure, the catalytic material 11.1 of the first filter section 6.1 is different from the catalytic material 11.2 of the second filter section 6.2.

The gas sensing device 1 shown in Figures 8 and 9 comprises four filter sections 6.1 to 6.4 arranged on the sensor chip 2. The four gas sensing elements 4 (of which only the gas sensing elements 4.1 and 4.2 are visible in Figure 8) comprise the same active sensing materials. However, the catalytic materials 6.1 to 6.4 of the filter sections 6.1 to 6.4 differ from each other.

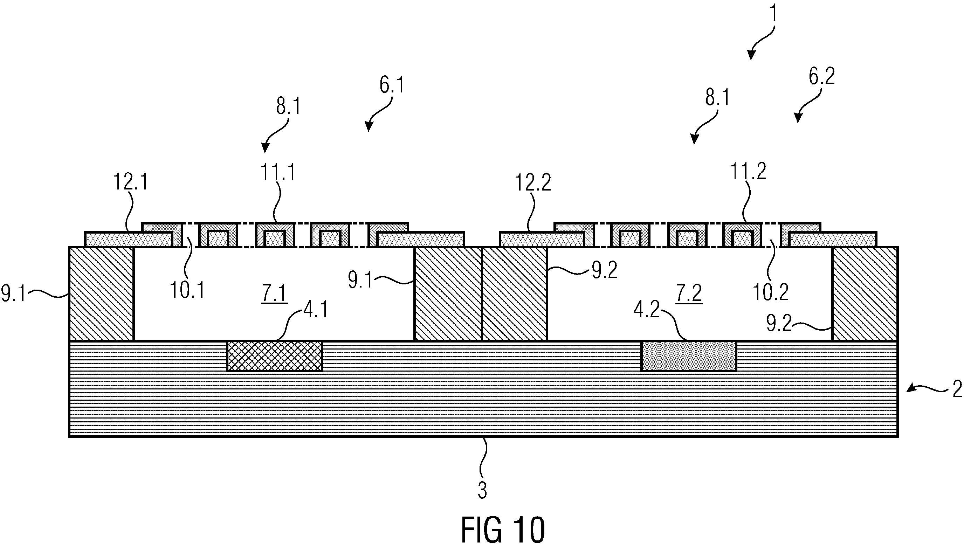

Figure 10 illustrates a seventh embodiment of a gas sensing device according to the invention in a schematic sectional side view.

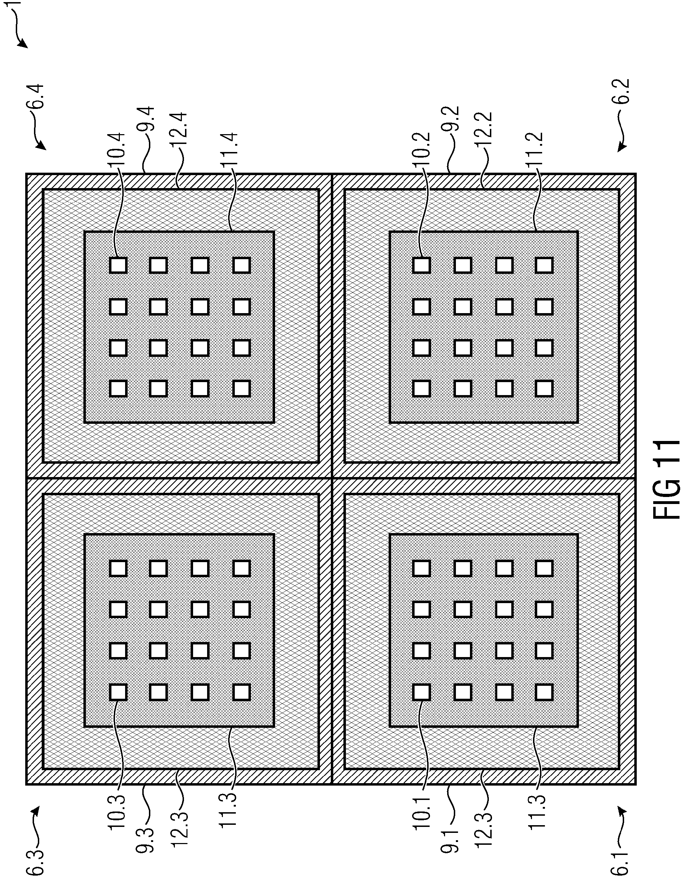

Figure 11 illustrates the seventh embodiment of a gas sensing device according to the invention in a schematic top view.

The seventh embodiment of the gas sensing device 1 is based on the sixth embodiment of the gas sensing device 1. In the following, only the different features of the seventh embodiment of the gas sensing device 1 are discussed.

According to embodiments of the disclosure, a sensing material of the first chemo-resistive gas sensing element 4.1 is different from a sensing material of the second chemo-resistive gas sensing element 4.2.

The gas sensing device 1 shown in Figures 10 and 11 comprises four filter sections 6.1 to 6.4 arranged on the sensor chip 2. The four gas sensing elements 4 (of which only the gas sensing elements 4.1 and 4.2 are visible in Figure 8) comprise active sensing materials, which are different from each other. However, the catalytic materials 6.1 to 6.4 of the filter sections 6.1 to 6.4 are identical.

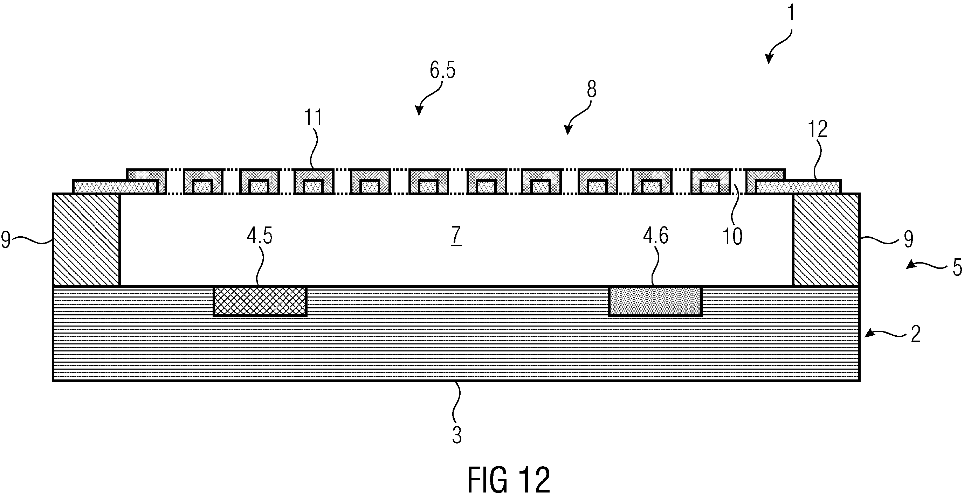

Figure 12 illustrates an eighth embodiment of a gas sensing device according to the invention in a schematic sectional side view.

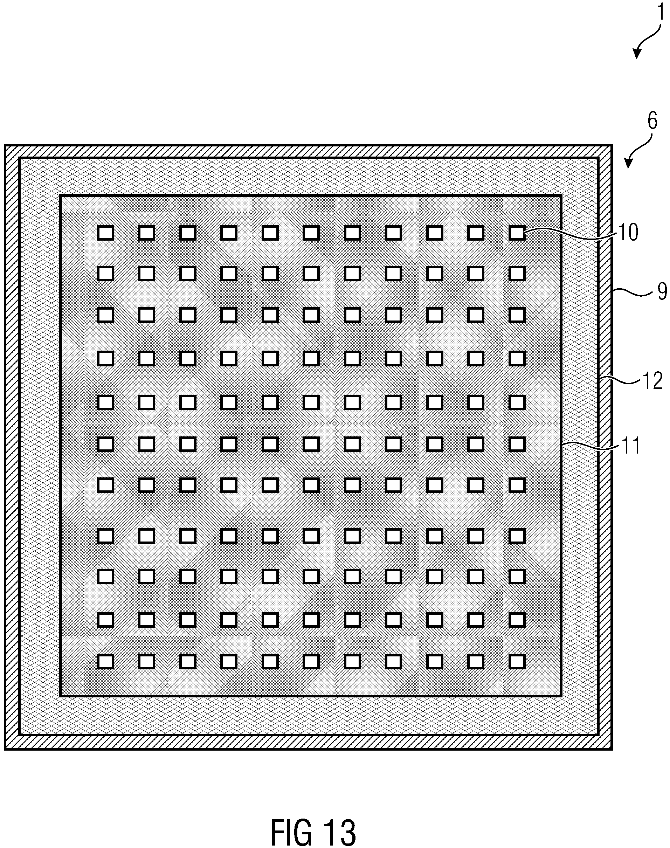

Figure 13 illustrates the eighth embodiment of a gas sensing device according to the invention in a schematic top view.

The eighth embodiment of the gas sensing device 1 is based on the first embodiment of the gas sensing device 1. In the following, only the different features of the eighth embodiment of the gas sensing device 1 are discussed.

According to embodiments of the disclosure, the sensor chip 2 comprises a fifth chemo-resistive gas sensing element 4.5 of the one or more chemo-resistive gas sensing elements 4 and a sixth chemo-resistive gas sensing element 4.6 of the one or more chemo-resistive gas sensing elements 4, wherein the catalytic gas filter arrangement 5 comprises a fifth filter section 6.5 of the one or more filter sections 6, wherein the fifth filter section 6.5 is arranged for filtering the ambient mixture of the plurality of gases for the fifth chemo-resistive gas sensing element 4.5 and for the sixth chemo-resistive gas sensing element 4.6.

According to embodiments of the disclosure, a sensing material of the fifth chemo-resistive gas sensing element 4.5 is different from a sensing material of the sixth chemo-resistive gas sensing element 4.6.

The gas sensing device 1 shown in Figures 12 and 13 comprises one filter section 6.5 arranged on the sensor chip 2. Both of a gas sensing element 4.5 and a gas sensing element 4.6 are arranged in the cavity 7 of the filter section 6.5. The sensing material of the gas sensing element 4.5 differs from the sensing material of the sensing element 4.6.

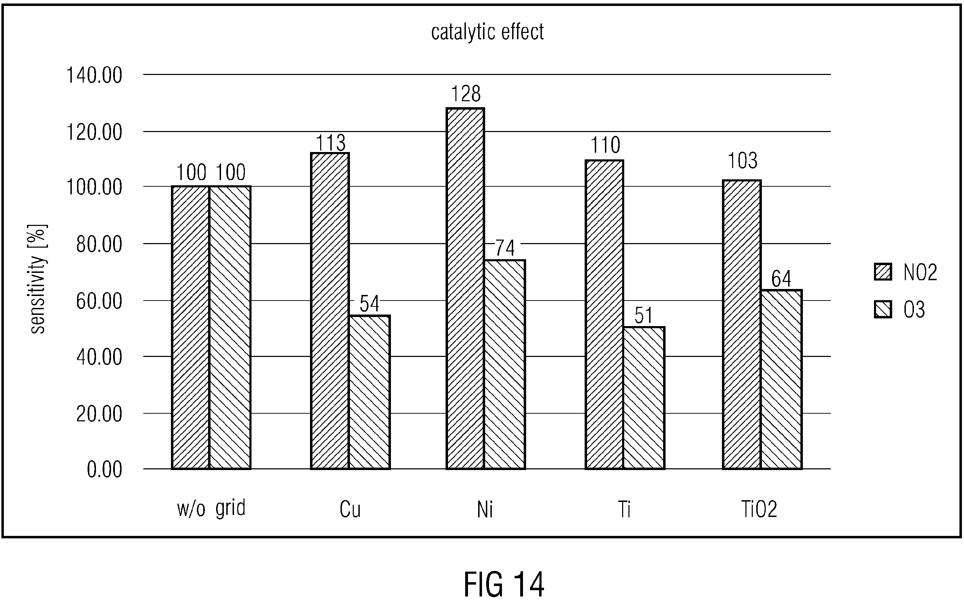

Figure 14 illustrate the catalytic effects of different catalytic materials. An evaluation of the proposed solution was done with four membranes 8 comprising different catalytic materials 11, namely Cu, Ni, Ti and TiO2. The membranes 8 had a diameter of 3.05 mm and a pore size of 38 µm. The membranes 8 were manually fixed on a 3D printed support structure 9. As a reference, a measurement without any membrane 8 was conducted.

The investigated membranes 8 have different impact on the sensitivity of the gas sensing device 1 depending on the type of gas. While the NO2 is practically not affected by them, the sensitivity of O3 drops significantly. This experiment suggests that the chosen materials effectively catalytically decompose O3 in-situ and reduce the amount of molecules that actually adsorb on the chemo-resistive gas sensing element 4.

The above described is merely illustrative, and it is understood that modifications and variations of the arrangements and the details described herein will be apparent to others skilled in the art. It is the intent, therefore, to be limited only by the scope of the impending claims and not by the specific details presented by way of description and explanation above.

### References:

[1] Batekliev et al., Ozone decomposition, Interdiscip Toxicol. 2014; Vol. 7(2): 47-59.

## Claims

1. A gas sensing device for sensing one or more gases to be sensed in an ambient mixture of a plurality of gases, the gas sensing device (1) comprising:
a sensor chip (2) comprising a substrate (3) and one or more chemo-resistive gas sensing elements (4) attached to the substrate (3), wherein each of the gas sensing elements (4) is configured for providing a sensor signal depending on at least one gas of the one or more gases to be sensed; and
a catalytic gas filter arrangement (5) comprising one or more filter sections (6), wherein each of the filter sections (6) comprises a cavity (7) which is covered with at least one membrane (8), wherein the at least one membrane (8) is supported by a support structure (9), wherein the at least one membrane (8) comprises gas permeable pores (10), wherein a surface defining the pores (10) comprises a catalytic material (11) for degrading at least one of the gases of the plurality of the gases;
wherein the gas filter arrangement (5) is arranged in such way that at least one of the chemo-resistive gas sensing elements (4) is exposed to a filtered mixture of the plurality of gases in the cavity (7) of one of the filter sections (6), wherein the filtered mixture of the plurality of gases is obtained by filtering the ambient mixture of the plurality of gases with the one of the filter sections (6).

2. The gas sensing device according to claim 1, wherein the at least one membrane (8) of one or more of the one or more filter sections (6) comprise a semiconductor bearing layer (12) being coated with the catalytic material (11).

3. The gas sensing device according to claim 1 or 2, wherein the support structure (9) of one of the filter sections (6) comprises one or more layers (13) of the sensor chip (2), so that the cavity (7) of the one of the filter sections is defined by the sensor chip (2) and by the at least one membrane (7) of the one of the filter sections (6).

4. The gas sensing device according to one of the claims 1 to 3, wherein at least the support structure (9) of one of the filter sections (6) comprises a plate-shaped semiconductor chip (14) having a through hole (15).

5. The gas sensing device according to claim 4, wherein the plate-shaped semi-conductor chip (14) of one of the filter sections (6) is attached to the sensor chip (2), so that the cavity (7) of the one of the filter sections (6) is defined by the through hole (15) of the plate-shaped semiconductor chip (14), by the sensor chip (2) and by the at least one membrane (8) of the one of the filter sections (6).

6. The gas sensing device according to claim 4, wherein the plate-shaped semi-conductor chip (14) of one of the filter sections (6) is attached to a housing (16) of the gas sensing device (1), so that the cavity (7) of the one of the filter sections (6)is defined by the housing (16) and by the at least one membrane (8) of the one of the filter sections (6).

7. The gas sensing device according to one of the claims 3 or 5, wherein the gas sensing device (1) comprise one or more particle filters (18) for preventing a penetration of particles into the cavity (7) of one or more of the one or more filter sections (6).

8. The gas sensing device according to claim 7, wherein the one or more particle filters (18) are attached to a housing (16) of the gas sensing device (1).

9. The gas sensing device according to claim 6, wherein the gas sensing device (1) comprise one or more particle filters (18) for preventing a penetration of particles into the cavity (7) of one or more of the one or more filter sections (6).

10. The gas sensing device according to claim 9, wherein the one or more particle filters (18) are attached to the housing (16) of the gas sensing device (1).

11. The gas sensing device according to one of the claims 1 to 10, wherein the sensor chip (2) comprises a first chemo-resistive gas sensing element (4.1) of the one or more chemo-resistive gas sensing elements (4) and a second chemo-resistive gas sensing element (4.2) of the one or more chemo-resistive gas sensing elements (4), wherein the catalytic gas filter arrangement (5) comprises a first filter section (6.1) of the one or more filter sections (6) and a second filter section (6.2) of the one or more filter sections (6), wherein the first filter section (6.1) is arranged for filtering the ambient mixture of the plurality of gases for the first chemo-resistive gas sensing element (4.1), and wherein the second filter section (6.2) is arranged for filtering the ambient mixture of the plurality of gases for the second chemo-resistive gas sensing element (4.).

12. The gas sensing device according to claim 11, wherein a sensing material of the first chemo-resistive gas sensing element (4.1) is different from a sensing material of the second chemo-resistive gas sensing element (4.2).

13. The gas sensing device according to one of the claims 11 or 12, wherein the catalytic material (11.1) of the first filter section (6.1) is different from the catalytic material (11.2) of the second filter section (6.2).

14. The gas sensing device according to one of the claims 1 to 13, wherein the sensor chip (2) comprises a fifth chemo-resistive gas sensing element (4.5) of the one or more chemo-resistive gas sensing elements (4) and a sixth chemo-resistive gas sensing element (4.6) of the one or more chemo-resistive gas sensing elements (4), wherein the catalytic gas filter arrangement (5) comprises a fifth filter section (6.5) of the one or more filter sections (6), wherein the fifth filter section (6.5) is arranged for filtering the ambient mixture of the plurality of gases for the fifth chemo-resistive gas sensing element (4.5) and for the sixth chemo-resistive gas sensing element (4.6).

15. The gas sensing device according to claim 14, wherein a sensing material of the fifth chemo-resistive gas sensing element (4.5) is different from a sensing material of the sixth chemo-resistive gas sensing element (4.6).

16. The gas sensing device according to one of the claims 1 to 15, wherein in a cross-sectional view the pores (10) of the one or more membranes (8) have a maximum extension smaller than 1000 nm and a minimum extension greater than 20 nm.

17. The gas sensing device according to one of the claims 1 to 16, wherein in a longitudinal view the pores (10) of the one or more membranes (8) have a length smaller than 2000 nm and greater than 40 nm.
